(19) Europäisches Patentamt
European Patent Office
Office européen des brevets

(11) **EP 2 026 769 B1**

(12) **EUROPEAN PATENT SPECIFICATION**

(45) Date of publication and mention
of the grant of the patent:
**17.03.2010 Bulletin 2010/11**

(21) Application number: **07725528.9**

(22) Date of filing: **24.05.2007**

(51) Int Cl.:
*A61K 9/24* (2006.01)    *A61K 9/32* (2006.01)
*A61K 9/36* (2006.01)    *A61K 31/366* (2006.01)
*A61K 31/401* (2006.01)    *A61K 31/525* (2006.01)
*A61K 31/519* (2006.01)    *A61P 9/10* (2006.01)
*A61P 9/12* (2006.01)

(86) International application number:
**PCT/EP2007/004631**

(87) International publication number:
**WO 2007/134870 (29.11.2007 Gazette 2007/48)**

(54) **BILAYER TABLET FOR PREVENTING CARDIOVASCULAR EVENTS**

ZWEISCHICHTIGE TABLETTE ZUR VERHINDERUNG VON KARDIOVASKULÄREN EREIGNISSEN

COMPRIMÉ BICOUCHE POUR LA PRÉVENTION D'ÉVÉNEMENTS CARDIOVASCULAIRES

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **24.05.2006 ES 200601355**

(43) Date of publication of application:
**25.02.2009 Bulletin 2009/09**

(73) Proprietor: **FERRER INTERNACIONAL, S.A.
08028 Barcelona (ES)**

(72) Inventors:
• **GUERRERO, Marta**
**E-08024 Barcelona (ES)**
• **ORRIOLS, Anna**
**E-08242 Manresa (ES)**

• **GUGLIETTA, Antonio**
**E-08750 Molins De Rei (ES)**

(74) Representative: **Reitstötter - Kinzebach
Patentanwälte
Sternwartstrasse 4
81679 München (DE)**

(56) References cited:
**WO-A-01/76632          WO-A-03/020243
WO-A-2005/011586      US-A1- 2003 049 314
US-A1- 2005 054 731**

• **NEUTEL J M: "LOW-DOSE ANTIHYPERTENSIVE
COMBINATION THERAPY: ITS RATIONALE AND
ROLE IN CRDIOVASCULAR RISK
MANAGEMENT" AMERICAN JOURNAL OF
HYPERTENSION, NEW YORK, NY, US, vol. 12, no.
8, 1999, pages 73S-79S, XP000866553**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the art

The invention relates to pharmaceutical compositions for preventing stroke in high-risk patients. More specifically, the invention relates to a bilayer tablet comprising a combination of a simvastatin compound, a lisinopril compound and a folic acid compound.

### State of the art

Cardiovascular disease is the main cause of mortality and morbidity in the developed world and it is becoming the main cause of mortality and morbidity worldwide. Although cardiovascular risk factors are well known, control thereof is below optimal, even in the most developed countries. Without a doubt, changes in eating habits and lifestyle reduce cardiovascular risk. Nevertheless, not enough work has been carried out to identify the most efficient and cost-effective way of implementing these changes in lifestyle. On the other hand, treatment and prevention of cardiovascular disease are expensive and their cost is increasing.

The low compliance with prescribed and self-administered treatments is well known to be a great problem in treating chronic diseases, such as in the case of cardiovascular disease. Compliance with chronic treatments is estimated at 50% in developed countries. The implications of the poor compliance with treatments are important both for the individual and for society. For the patient, treatment benefits are reduced, producing infra-treatment and making it more difficult for the clinician to evaluate the effectiveness and to determine the best dosage for such treatments. For society, the poor compliance leads to generating chemical waste, the increase in health costs and self-medication.

The complexity of dosages and adverse effects are the factors related to the drug which most affect prescription compliance. These two factors rapidly increase with the use of multiple therapies to treat the disease or with the treatment of more than one disease in the same patient, giving rise to even less compliance with the medication.

In this context, Wald and Law (Br. Med. J. 326, No. 7404, 1419-23, 2003) conceived the term polypill referring to the hypothetic combination of drugs such as statins, antihypertensive drugs, aspirin and vitamins, such as folic acid, to be used in a single daily tablet. These authors recommended the use of the polypill as a way to prevent complications of cardiovascular disease with minimal adverse reactions based on meta-analyses carried out with short duration clinical trials. Thus, cardiovascular prevention based on the polypill containing several components that are effective in treating cardiovascular risk factors would prevent a high proportion of ischaemic cardiopathies and ictus. They concluded that the polypill strategy would be safe and that its generalized use would have a greater impact in cardiovascular disease prevention in occidental countries than any other intervention and they estimated that the polypill could reduce the incidence of coronary disease and ictus in up to 88 and 80%, respectively.

The authors analyzed the most suitable patients for taking the polypill. The candidates would be patients who had suffered an acute coronary syndrome or an ischaemic ictus, patients with stable chronic angina, transitory ischaemic episodes and diabetics patients. Among the population without prior cardiovascular disease, the most determining factor is age. Since 96% of deaths by acute coronary syndrome or ictus occur in people over 55 years of age, preventive treatment for people over 55 years of age would prevent almost all of these deaths. That is, the best strategy would be to treat all patients with ischaemic disease and all people over 55 years of age.

Several patents have been published in this direction regarding polypills for cardiovascular prevention.

The patent US6576256 covers the combination of a hypocholesterolemic agent, an inhibitor of the renin-angiotensin system, aspirin and at least one vitamin chosen amongst vitamin B6, vitamin B12 and folic acid.

Patent application WO0115674 covers the use of an inhibiting agent for the renin-angiotensin system, a lipid-lowering agent, a diuretic agent and aspirin.

Patent application WO0176632 covers the combination of hydrochlorotiazide, atenolol and enalapril as antihypertensive agents, atorvastatin as a lipid-regulating agent, aspirin as a platelet anti-aggregating agent and folic acid in order to reduce serum levels of homocysteine.

Patent application WO03020243 covers the combination of a lipid-lowering agent, a renin-angiotensin inhibitor and aspirin.

Patent application WO2004080488 covers the combination of aspirin, an HMG CoA reductase inhibitor and an antihypertensive substance.

Patent application WO2005011586 covers the combination of an antagonist to β-adrenergic receptors or a diuretic agent, or both, a hypocholesterolemic agent, a renin-angiotensin system inhibitor and aspirin.

Patent application WO2005025673 covers a combination of a hypoglycemiant agent of the biguanide family, a lipid-lowering agent and a hypertensive agent.

On the other hand, simvastatin is one of the most widely prescribed HMG CoA reductase inhibitor in hypercholesterolemia therapy. It further has the advantage over other compounds in its family that its impurities are well known both qualitatively and quantitatively.

Similarly, lisinopril is one of the most widely prescribed angiotensin-converting enzyme inhibitor for hypertension therapy. From the pharmacotechnical point of view it has the advantage over other compounds in its family that it is obtained with less impurities and that it is one of the most soluble in water, which leads to greater ease of absorption.

In turn, there is growing evidence that the high levels of homocysteine are associated to an increased risk of ischaemic coronary and cerebrovascular disease. In this sense, folic acid is considered a regulator or normalizer of the high values of homocysteine. The association mechanism is unknown, but it is well known that folic acid reduces plasma levels of homocysteine by increasing its catabolism.

There is no article referring to the combination of these three active ingredients, either at a pharmacological or clinical level, understanding as combination both the administration of all three components in a single dosage form and the concomitant administration of the three components in several forms. There are not any patents illustrating said combination, either the formulation of the three compounds as single active ingredients in a single dosage form or the concomitant administration of the three compounds individually.

Thus, it seems advisable to provide a combination of simvastatin, lisinopril and folic acid comprising an optimal dose of the three active ingredients in a single dosage form for the prevention of stroke in the high-risk population. Specifically, high-risk populations are people above 55 years of age, patients with a history of angina pectoris, ictus, arteriosclerosis, intermittent claudication, diabetes coronary disease peripheral vascular disease, altered platelet function, hemodialysis, hypercholesterolemia, hypertension, myocardial infarction, congestive heart failure, ischaemia, nephropathy, high serum homocysteine levels, cardiac arrest or restenosis, smokers, obese and sedentary populations.

**Brief description of the drawings**

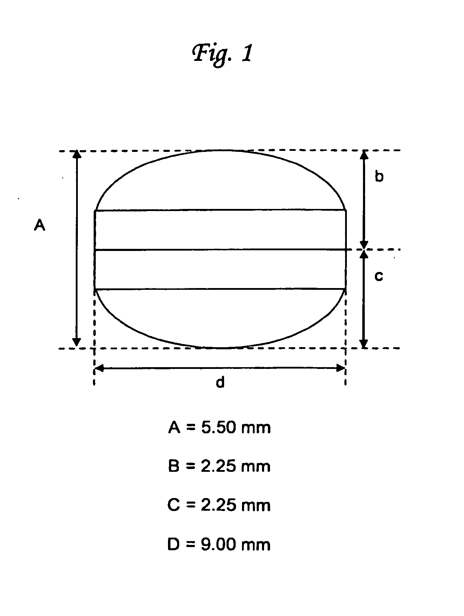

Figure 1 shows the standard shape and dimensions of a 360 mg bilayer tablet object of the present invention.

Figure 2 shows the *in vitro* release profile for each one of the active ingredients of the bilayer tablet obtained by direct compression and of the active ingredients separately corresponding to each compartment.

Figure 3 shows the *in vitro* release profile for each one of the active ingredients of the bilayer tablet obtained by wet granulation and of the active ingredients separately corresponding to each compartment.

**Detailed description of the invention**

The present invention solves the aforementioned need for a combination of simvastatin, lisinopril and folic acid in a single dosage form at optimal doses, by providing a bilayer tablet comprising the following two compartments:

- a compartment (i) comprising as an active ingredient a pharmaceutically acceptable simvastatin compound; and

- a compartment (ii) comprising as active ingredients a pharmaceutically acceptable lisinopril compound and pharmaceutically acceptable folic acid compound;

characterized by the fact that both compartments are isolated from one another.

In a preferred aspect of the present invention, the pharmaceutically acceptable simvastatin compound used is free simvastatin, the pharmaceutically acceptable lisinopril compound is chosen indistinctly from free lisinopril and lisinopril dihydrate and the pharmaceutically acceptable folic acid compound is chosen indistinctly from free folic acid and folic acid dihydrate. Preferably the folic acid compound is free folic acid.

The amount of simvastatin used in each tablet is present in an amount comprised between 2.5 and 20 mg, both inclusive, preferably between 5 and 10 mg; the lisinopril compound is present in an amount comprised between 1 and 10 mg, both inclusive, preferably between 2.5 and 5 mg, and the free folic acid is present in an amount comprised between 0.1 and 1 mg, both inclusive, preferably between 0.2 and 0.5 mg, both inclusive.

Simvastatin is highly sensitive to light and moisture. For this reason in the tablet of the present invention this compound is isolated in a different compartment to that occupied by lisinopril and folic acid.

In a preferred aspect of the present invention, the tablet is obtained by direct compression.

In a preferred aspect of the present invention, the tablet is obtained by wet granulation.

In a preferred aspect of the present invention, when the tablet is obtained by direct compression it comprises the following suitable excipients:

- for compartment (i) a disintegrant agent, a glidant agent, a diluent agent, a lubricant agent and a release agent; and

- for compartment (ii) a diluent agent, a disintegrant agent, a glidant agent and a release agent.

The disintegrant agent of compartment (i) is chosen from croscarmellose sodium, sodium starch glycolate, crospovidone, sodium lauryl sulphate, several forms of microcrystalline cellulose such as microcrystalline cellulose PH 101 and microcrystalline cellulose PH 102, and the like, and mixtures thereof, such that the total weight of the disintegrant agents is comprised between 2 and 10% of the compartment weight, both inclusive.

The glidant agent of compartment (i) is chosen from colloidal anhydrous silica, corn starch, talc, magnesium trisilicate, and the like, and mixtures thereof, such that the total weight of the glidant agents is comprised between 0.1 and 10% of the compartment weight, both inclusive.

The diluent agent of compartment (i) is chosen from silicified microcrystalline cellulose, several microcrystalline cellulose forms such as microcrystalline cellulose PH 101 and microcrystalline cellulose PH 102, anhydrous lactose, hydrated lactose, calcium phosphate, dicalcium phosphate, tricalcium phosphate, mannitol, sorbitol, sucrose, inositol, trehalose, xylitol, corn starch, kaolin, bentonite, and the like, and mixtures thereof, such that the total weight of the diluent agents is comprised between 20 and 90% of the compartment weight both inclusive.

The lubricant agent of compartment (i) is chosen from talc, sodium benzoate, poloxamer and similar agents and mixtures thereof, such that the total weight of the agents is comprised between 1 and 10% of the compartment weight, both inclusive.

The release agent of compartment (i) is chosen from magnesium stearate, sodium stearyl fumarate, calcium stearate, zinc stearate, stearic acid, polyethylene glycols with a molecular weight of $\geq$ 6000, and the like, and mixtures thereof, such that the total weight of the release agents is comprised between 0.25 and the 5% of the compartment weight, both inclusive.

The diluent agent of compartment (ii) is chosen from silicified microcrystalline cellulose, several microcrystalline cellulose forms such as microcrystalline cellulose PH 101 and microcrystalline cellulose PH 102, anhydrous lactose, hydrated lactose, calcium phosphate, dicalcium phosphate, tricalcium phosphate, mannitol, sorbitol, sucrose, inositol, trehalose, xylitol, corn starch, kaolin, bentonite, and the like, and mixtures thereof, such that the total weight of the diluent agents is comprised between 20 and 95% of the compartment weight both inclusive.

The glidant agent of compartment (ii) is chosen from colloidal anhydrous silica, corn starch, talc, magnesium trisilicate, and the like, and mixtures thereof, such that the total weight of the glidant agents is comprised between 0.1 and 10% of the compartment weight, both inclusive.

The disintegrant agent of compartment (ii) is chosen from croscarmellose sodium, sodium starch glycolate, crospovidone, sodium lauryl sulphate, several forms of microcrystalline cellulose such as microcrystalline cellulose PH 101 and microcrystalline cellulose PH 102, and the like, and mixtures thereof, such that the total weight of the disintegrant agents is comprised between 2 and 5% of the compartment weight, both inclusive.

The release agent of compartment (ii) is chosen from magnesium stearate, sodium stearyl fumarate, calcium stearate, zinc stearate, stearic acid, polyethylene glycols with a molecular weight of $\geq$6000, and the like, and mixtures thereof.

In a preferred aspect of the present invention, when the tablet is obtained by wet granulation it comprises the following suitable excipients:

- for compartment (i) a disintegrant agent, a glidant agent, a diluent agent, a binding agent, a solubilizing agent, a lubricant agent and a release agent; and

- for compartment (ii) a disintegrant agent, a glidant agent, a diluent agent, a binding agent and a release agent.

The disintegrant agent of compartment (i) is chosen from crospovidone, croscarmellose sodium, sodium starch glycolate, sodium lauryl sulphate, several forms of microcrystalline cellulose such as microcrystalline cellulose PH 101 and microcrystalline cellulose PH 102, and the like, and mixtures thereof, such that the total weight of the disintegrant agents is comprised between 2 and 10% of the compartment weight, both inclusive.

The glidant agent of compartment (i) is chosen from colloidal anhydrous silica, corn starch, talc, magnesium trisilicate, and the like, and mixtures thereof, such that the total weight of the glidant agents is comprised between 0.1 and 10% of the compartment weight, both inclusive.

The diluent agent of compartment (i) is chosen from silicified microcrystalline cellulose, several microcrystalline cellulose forms such as microcrystalline cellulose PH 101 and microcrystalline cellulose PH 102, anhydrous lactose, hydrated lactose, calcium phosphate, dicalcium phosphate, tricalcium phosphate, mannitol, sorbitol, sucrose, inositol, trehalose, xylitol, corn starch, kaolin, bentonite, and the like, and mixtures thereof, such that the total weight of the diluent agents is comprised between 20 and 90% of the compartment weight, both inclusive.

The binding agent of compartment (i) is chosen from povidone k-30, hydroxypropylmethylcellulose, carboxymethylcellulose, pregelatinized starch, corn starch paste, and the like, and mixtures thereof, such that the total weight of the binding agents is comprised between 0.5 and 20% of the compartment weight, both inclusive.

The solubilizing agent of compartment (i) is chosen from sodium lauryl sulphate, polysorbate 80, lauroyl macrogol-32 glycerides, and the like, and mixtures thereof, such that the total weight of the agents is comprised between 0.1 and 3% of the compartment weight, both inclusive.

The lubricant agent of compartment (i) is chosen from talc, sodium benzoate, poloxamer, and the like, and mixtures thereof, such that the total weight of the agents is comprised between 1 and 10% of the compartment weight, both inclusive.

The release agent of compartment (i) is chosen from magnesium stearate, sodium stearyl fumarate, calcium stearate, zinc stearate, stearic acid, polyethylene glycols with a molecular weight of $\geq 6000$, and the like, and mixtures thereof, such that the total weight of the release agents is comprised between 0.25 and 5% of the compartment weight, both inclusive.

The disintegrant agent of compartment (ii) is chosen from crospovidone, croscarmellose sodium, sodium starch glycolate, sodium lauryl sulphate, several forms of microcrystalline cellulose such as microcrystalline cellulose PH 101 and microcrystalline cellulose PH 102, and the like, and mixtures thereof, such that the total weight of the disintegrant agents is comprised between 2 and 5% of the compartment weight, both inclusive.

The glidant agent of compartment (ii) is chosen from colloidal anhydrous silica, corn starch, talc, magnesium trisilicate, and the like, and mixtures thereof, such that the total weight of the glidant agents is comprised between 0.1 and 10% of the compartment weight, both inclusive.

The diluent agent of compartment (ii) is chosen from microcrystalline cellulose PH 101, microcrystalline cellulose PH 102, and the like, and mixtures thereof, such that the total weight of the diluent agents is comprised between 20 and 50% of the compartment weight, both inclusive.

The binding agent of compartment (ii) is chosen from povidone k-30, hydroxypropylmethylcellulose, carboxymethylcellulose, pregelatinized starch, corn starch paste, and the like, and mixtures thereof, such that the total weight of the binding agents is comprised between 0.5 and the 20% of the compartment weight, both inclusive.

The release agent of compartment (ii) is chosen from magnesium stearate, sodium stearyl fumarate, calcium stearate, zinc stearate, stearic acid, polyethylene glycols with a molecular weight of $\geq 6000$, and the like, and mixtures thereof, such that the total weight of the release agents is comprised between 0.25 and 5% of the compartment weight, both inclusive.

The aforementioned glidant agents are chosen such that colloidal anhydrous silica may vary between 0.1 and 1.0%, corn starch between 1.0 and 10.0%, talc between 1.0 and 10.0%, and magnesium trisilicate between 0.1 and 1.0%, such that the total weight of the glidant agents is comprised between 0.1 and 10%, both inclusive.

The aforementioned lubricant agents are chosen from talc, the proportion of which may vary between 1.0 and 10.0%, sodium benzoate, the proportion of which may vary between 2.0 and 5.0%, and poloxamer, such that the total weight of the lubricant agents is comprised between 1 and 10%, both inclusive.

The aforementioned release agents are chosen such that magnesium stearate may vary between 0.25 and 5.0%, sodium stearyl fumarate between 0.5 and 2%, calcium stearate between 0.5 and 1.0%, zinc stearate between 0.5 and 1.5%, stearic acid between 1.0 and 3.0%, and polyethylene glycols of a molecular weight of $\geq 6000$ between 0.25 and 5.0%, such that the total weight of the release agents is comprised between 0.25 and 5%, both inclusive.

The aforementioned binding agents are chosen such that povidone may vary between 0.5 and 5.0%, hydroxypropylmethylcellulose between 2.0 and 5.0%, sodic carboxymethylcellulose between 1.0 and 6.0%, pregelatinized starch between 5.0 and 10.0%, and corn starch paste between 10.0 and 20.0%, such that the total weight of the binding agents is comprised between 0.5 and 20%, both inclusive.

The aforementioned solubilizing agents are chosen such that sodium lauryl sulphate may vary between 1.0 and 2.0%, polysorbate 80 (Tween® 80, Quimica Massó, Barcelona, Spain) between 0.1 and 3.0%, and of lauroyl macrogol-32 glycerides (Gelucire® 44/14, Gattefosse SA, Saint Priest, France) between 0.5 and 1.0%, the total weight of the solubilizing agents is comprised between 0.1 and 3%, both inclusive.

In a preferred aspect of the present invention, the tablet further comprises a protecting coating that protects it from light and moisture. Said coating is performed by means of coating-forming polymers, to be chosen from between an acrylic polymer and a cellulose derivative. When the acrylic polymer is used, this is the basic butylated methacrylate copolymer. When the cellulose derivative is used, it is chosen from hydroxypropylmethylcellulose, hydroxypropylcellulose, methylcellulose, hydroxyethylcellulose and mixtures thereof. It may optionally comprise one or more lubricants, one or more plasticizers, and one or more opacifiers.

The protective coating against light and moisture is performed by means of coating polymers. Coating polymers suitable for the tablets of the present invention are acrylic polymers of the Eudragit® type, preferably an immediate release polymer such as Eudragit® EPO (basic butylated methacrylate copolymer), cellulose derivatives, to be chosen between hydroxypropylmethylcellulose, hydroxypropylcellulose, methylcellulose, hydroxyethylcellulose, and the like, and mixtures thereof, the mixtures of said coating cellulose polymers with lubricants and opacifiers, or mixtures of such polymers with plasticizers and optionally with opacifiers, ready-made and prepared for dispersing and applying, such as Opadry® II (Colorcon, West Point, USA), Sepifilm® LP (Seppic, Paris, France), and the like, agents and mixtures thereof.

In a preferred aspect of the present invention, the resulting tablet has a total weight comprised between 150 and 400 mg, both inclusive, preferably between 180 and 380 mg.

In a preferred aspect of the present invention, the tablet is used for the manufacture of a drug for the prevention of stroke in high-risk conditions or diseases.

In a more preferred aspect of the present invention, the tablet is used in high-risk conditions such as being older than 55 years of age or diseases such as angor pectoris, ictus, arteriosclerosis, intermittent claudication, diabetes, coronary disease, peripheral vascular disease, altered platelet function, hemodialysis, hypercholesterolemia, hypertension, myocardial infarction, congestive heart failure, ischaemia, nephropathy, high serum homocysteine levels, cardiac arrest or restenosis, smoking, obesity and a sedentary lifestyle.

A preferred aspect of the present invention is to provide a method for the prevention of stroke in high-risk conditions or diseases comprising the administration of the tablet disclosed.

A more preferred aspect of the present invention is to contribute a method for the prevention of stroke in which high-risk conditions or diseases are being older than 55 years of age, angor pectoris, ictus, arteriosclerosis, intermittent claudication, diabetes, coronary disease, peripheral vascular disease, altered platelet function, hemodialysis, hypercholesterolemia, arterial hypertension, myocardial infarction, congestive heart failure, ischaemia, nephropathy, high serum homocysteine levels, cardiac arrest or restenosis, smoking, obesity and a sedentary lifestyle, comprising the administration of the tablet disclosed.

## Embodiments of the invention

The present invention is additionally illustrated by means of the following examples, which do not intent to limit the scope thereof.

**Example 1:** Formulations of bilayer tablets

The following excipients were used to manufacture the formulations:

Microcrystalline cellulose PH 101, Avicel® - FMC, Barcelona, Spain;
Microcrystalline cellulose PH 102, Avicel® - FMC, Barcelona, Spain;
silicified microcrystalline cellulose, Prosolv® HD 90 - JRS Pharma, Riegate, Surrey, UK;
Croscarmellose sodium, Ac-Di-Sol® - FMC, Philadelphia, USA;
Crospovidone, Polyplasdone® - ISP Technologies Inc, Calvert City, KY, USA;
Lauroyl macrogol-32 glycerides, Gelucire® 44/14 - Gattefosse SA - Saint Priest, France;
Povidone k-30, Plasdone® k29-32 - ISP Technologies Inc, Texas City, TX, USA; and
Colloidal anhydrous silica, Aerosil® 200 - Degussa Corporation, Persippany, USA.

a) Formulation manufactured by direct compression of both compartments

*Composition of the first Compartment (A)*

|  | Amount (%) | Amount (mg) | Function |
|---|---|---|---|
| Simvastatin | 5.55 | 10.0 | Active ingredient |
| Ac-Di-Sol® (croscarmellose sodium) | 5.0 | 9.0 | Disintegrant |
| Aerosil® 200 (colloidal anhydrous silica) | 0.55 | 1.0 | Glidant |
| Prosolv® HD 90 (silicified microcrystalline cellulose) | 84.9 | 152.8 | Diluent |
| Talc | 3.0 | 5.4 | Lubricant |
| Magnesium stearate | 1.0 | 1.8 | Release agent |
| **Total** | **100.0** | **180.0** | — |

*Manufacture of the First Compartment ( A )*

All the components of the formulation were individually weighed and sieved through a 0.5 mm diameter sieve except for magnesium stearate. They were mixed in a shaker-mixer and the mixture was set aside. The magnesium stearate was then sieved through a 0.5 mm sieve and added to the previous mixture in a shaker-mixer for 5 more minutes in order to obtain the final mixture for pressing.

The final mixture thus obtained was ready to be compressed in a Manesty F3 eccentric tablet press provided with punches of 9 mm of diameter, the nominal weight of the tablet being 180 mg. Nevertheless, in order to obtain the bilayer tablets said mixture was kept up to the manufacture of the second compartment ( B ).

*Composition of the Second Compartment ( B )*

|  | Amount (%) | Amount (mg) | Function |
|---|---|---|---|
| Lisinopril dihydrate | 2.77 | 5.0 | Active ingredient |
| Folic acid | 0.22 | 0.4 | Active ingredient |
| Avicel® PH 101 (Microcrystalline cellulose PH 101) | 45.68 | 82.2 | Diluent |
| Avicel® PH 102 (Microcrystalline cellulose PH 102) | 45.68 | 82.2 | Diluent |
| Aerosil® 200 (colloidal anhydrous silica) | 0.55 | 1.0 | Glidant |
| Ac-Di-Sol® (croscarmellose sodium) | 4.44 | 8.0 | Disintegrant |
| Magnesium stearate | 0.66 | 1.2 | Release agent |
| **Total** | **100.0** | **180.0** | — |

*Manufacture of the Second Compartment ( B )*

The previously weighed folic acid was dispersed in a mortar by means of progressive dilutions, adding fractions of 5% of the total microcrystalline cellulose in the formula, also previously weighed. The rest of the formula was weighed except for the magnesium stearate and it was added to the previous mixture. It was sieved through a 0.5 mm sieve and mixed in a shaker-mixer for 15 minutes.

Finally, the magnesium stearate was weighed and sieved through a 0.5 mm sieve and it was added to the previous mixture in a shaker-mixer for 5 more minutes.

The final mixture thus obtained was ready to be compressed in a Manesty F3 eccentric tablet press provided with punches of 9 mm of diameter, the nominal weight of the tablet being 180 mg. Nevertheless, in order to obtain the bilayer tablets said mixture was set aside to proceed to the manufacture of bilayer tablets together with the previously prepared mixture corresponding to the First Compartment (A).

*Manufacture of the bilayer tablets*

In order to manufacture the bilayer tablets, the amounts of powder required for each compartment (180 mg of mixture corresponding to the First Compartment (A) and 180 mg of mixture corresponding to the Second Compartment (B)) were weighed separately. The same Manesty F3 eccentric tablet press provided with concave punches of 9 mm diameter was used. The formulation of the lisinopril dihydrate and the folic acid (B) compartment was pressed manually by putting the mixture inside the matrix of the tablet press and then, without removing the tablet formed from the matrix and stepping back in the compression cycle, the remaining space in the matrix was filled with the formulation of the simvastatin compartment ( A ) and the cycle was completed until expelling the bilayer tablet. The tablets thus obtained weighed 360 mg, had a strength of 180-220 N and a reliability of less than 0.1% without lamination or separation of the two layers.

b) Formulation manufactured by wet granulation of both compartments

*Composition of the First Compartment ( C )*

|  | Amount (%) | Amount (mg) | Function |
|---|---|---|---|
| Micronized simvastatin[1] | 5.55 | 10.0 | Active ingredient |
| Polyplasdone® XL (crospovidone) | 5.0 | 9.0 | Disintegrant |
| Aerosil® 200 (colloidal anhydrous silica) | 0.55 | 1.0 | Glidant |
| Avicel® PH 101 (Microcrystalline cellulose PH 101) | 78.9 | 142.0 | Diluent |
| Plasdone® k29-32 (povidone k-30)[2] | 5.0 | 9.0 | Binder |
| Gelucire® 44/14[3] (Lauroyl macrogol-32 glycerides) | 1.0 | 1.8 | Solubilizer |
| Talc | 3.0 | 5.4 | Lubricant |
| Magnesium stearate | 1.0 | 1.8 | Release agent |
| **Total** | **100.0** | **180.0** | — |
| [1] Micronized raw material in a Rina-Jet. It is micronized in order to improve active ingredient solubility. [2] It is incorporated in the formulation as a binding solution at 10,0% in 96˚ Ethanol. [3] It is incorporated into the binding solution. | | | |

*Manufacture of the First Compartment ( C )*

Forming part of the intragranular components are the active ingredient (simvastatin), the disintegrant (crospovidone), the binder (povidone k-30), the solubilizer (Gelucire® 44/14) and 95% of the diluent (microcrystalline cellulose PH101). The extragranular excipients are the glidant (colloidal anhydrous silica), the lubricant and the release agent (talc and magnesium stearate) and the remaining 5% of the diluent (microcrystalline cellulose).

The intragranular components were weighed except the binder and the solubilizer. They were sieved through a 0.5 mm mesh and mixed in a shaker-mixer for 10 min. The mixture was placed in the mortar for subsequent mixing.

The binding solution required for the mixing was prepared in a separate container, the solubilizer (Gelucire® 44/14) also being added to this solution.

It was mixed and passed through the wet granulator with a 1.2 mm mesh and at 100 rpm. The granules obtained were left to dry for 20-24 h in a heater at 40˚C. Once this time had elapsed they were withdrawn from the heater and passed through the oscillating granulator with a 0.8 mm mesh.

Approximately 2 g were set apart to determine moisture in the thermobalance for 60 min at 90˚C.

The remaining granules were weighed in order to calculate the extragranular components necessary in order to complete the formula. Having calculated the amount of each one of the extragranular components they were all weighed and sieved through 0.5 mm except for magnesium stearate.

Intragranular granules and extragranular excipients were mixed in a shaker-mixer for 15 minutes.

The magnesium stearate was weighed and sieved through a 0.5 mm mesh and it was added to the previous mixture obtained, mixing it in a turbula shaker-mixer for 5 final minutes.

The final mixture obtained was ready to be compressed in a Manesty F3 eccentric tablet press provided with punches of 9 mm of diameter, the nominal weight of the tablet being 180 mg. Nevertheless, in order to obtain the bilayer tablets said mixture was kept up to the manufacture of the second compartment (D).

*Composition of the Second Compartment (D)*

|  | Amount (%) | Amount (mg) | Function |
|---|---|---|---|
| Lisinopril dihydrate | 2.77 | 5.0 | Active ingredient |
| Folic acid Folic acid [1] | 0.22 | 0.40 | Active ingredient |

(continued)

|  | Amount (%) | Amount (mg) | Function |
|---|---|---|---|
| Polyplasdone® XL (crospovidone) | 4.0 | 7.2 | Disintegrant |
| Aerosil® 200 (colloidal anhydrous silica) | 0.55 | 1.0 | Glidant |
| Avicel® PH 101 (Microcrystalline cellulose PH 101) | 43.4 | 78.1 | Diluent |
| Avicel® PH 102 (Microcrystalline cellulose PH 102) | 43.4 | 78.1 | Diluent |
| Plasdone® k29-32 (2) (povidone k-30) | 5.0 | 9.0 | Binder |
| Magnesium stearate | 0.66 | 1.2 | Release agent |
| **Total** | **100.0** | **180.0** | — |

(1) It is incorporated in the formulation through the binding solution in order to improve its uniformity in the final tablet due to its low dose.
(2) It is incorporated in the formulation as a binding solution at 5.0% (96° Ethanol: Purified water 50:50).

*Manufacture of the Second Compartment (D)*

Forming part of the intragranular excipients are lisinopril dihydrate, the disintegrant (crospovidone), the binder (povidone k-30) and 95% of the diluent (microcrystalline cellulose PH101 and PH102). The folic acid is incorporated to the binding solution with the purpose of obtaining a good distribution within the mixture and to form part of the intragranulate. Extragranular excipients are the glidant (colloidal anhydrous silica), the release agent (magnesium stearate) and the remaining 5% of the diluent (microcrystalline cellulose PH101 and PH102).

Intragranular components were weighted, sieved through a 0.5 mm mesh and homogenized in a shaker-mixer for 10 min. It was placed in a mortar for mixing.

The binding solution required for the mixing (with povidone k-30) was prepared in a separate container. The folic acid was dispersed in the binding solution and subsequently added with continuous magnetic stirring of said solution.

It was mixed and passed through the wet granulator with a 1.2 mm mesh and at 100 rpm. The granules obtained were left to dry for 20-24 h in a heater at 40°C. Once this time had elapsed they were withdrawn from the heater and passed through the oscillating granulator with a 0.8 mm mesh.

Approximately 2 g were set apart to determine moisture in the thermobalance for 60 min at 90°C.

The remaining granules were weighed in order to thus calculate the extragranular components necessary in order to complete the formula. Having calculated the amount of each one of the extragranular components they were all weighed and sieved through 0.5 mm except for magnesium stearate.

Intragranular granules and extragranular excipients were mixed in a turbula shaker-mixer for 15 minutes.

The magnesium stearate was weighed and sieved through a 0.5 mm sieve and it was added to the previous mixture obtained, mixing it in a shaker-mixer for 5 final minutes.

The mixture obtained was ready to be compressed in a Manesty F3 eccentric tablet press provided with punches of 9 mm of diameter, the nominal weight of the tablet being 180 mg. Nevertheless, in order to obtain the bilayer tablets said mixture was set aside to proceed to the manufacture of bilayer tablets together with the previously prepared mixture corresponding to the First Compartment (C).

*Manufacture of the bilayer tablets*

In order to manufacture the bilayer tablets, the amounts of powder required for each compartment (180 mg of mixture corresponding to the First Compartment (C) and 180 mg of mixture corresponding to the Second Compartment (D)) were weighed separately. The same Manesty F3 eccentric tablet press provided with concave punches of 9 mm diameter was used, The formulation of the lisinopril dihydrate compartment and the folic acid (D) was pressed manually by introducing the mixture inside the matrix of the tablet press and then, without removing the tablet formed from the matrix and stepping back in the compression cycle, the remaining space in the matrix was filled with the formulation of the simvastatin compartment (C) and the cycle was completed until expelling the bilayer tablet. The tablets obtained weighed 360 mg, a resistance to rupture of 180-220 N and a friability of lower than 0.1 % without lamination or separation of the two layers.

Deviations below 5% were obtained for both formulations in the theoretical value of the weight and dimensions of the tablet, determined according to the methodologies described in Examples 3.1 and 3.6, respectively.

Disgregation values were below 5 minutes for both formulations, determined according to the methodology of Example 3.4. Friability values of 0%, were obtained according to the methodology of Example 3.3. The two formulations showed a release exceeding 95% after 30 minutes. The assay and uniformity of content for each one of the active ingredients were between 90 and 110%, determined with the techniques described in Example 4.

Angles of repose were also determined for both mixtures, determined according to the methodology described in Example 3.7. It was determined that angles of repose of 25-30˚ implied excellent flow properties, whereas angles of 31-35˚ implied good flow properties, those of 36-40˚ were acceptable and for 41˚ onwards were unacceptable. The results are shown in Table 1.

Table 1

| Compartment | Slip velocity (s) | Angle of repose (˚) |
|---|---|---|
| ( A ) | 13.5 | 34.40 |
| ( B ) | 20.2 | 38.03 |
| ( C ) | 9.11 | 31.85 |
| ( D ) | 8.7 | 31.60 |

Compressibility factors and Hausner indices were also determined according to the methodology described in Example 3.9. Hausner indices of 1.26-1.34 and compressibility factors of 21-25% were considered acceptable, and values of 1.00-1.11 and 1-10%, respectively, were considered excellent. The results are shown in Table 2.

Table 2

| Compartment | Bulk density (g/mL) | Tapped density (g/mL) | Hausner Index | Compressibility factor |
|---|---|---|---|---|
| ( A ) | 0.51 | 0.61 | 1.20 | 16.39% |
| ( B ) | 0.40 | 0.52 | 1.29 | 23.08 % |
| ( C ) | 0.34 | 0.41 | 1.17 | 17.07% |
| ( D ) | 0.40 | 0.45 | 1.14 | 11.11 % |

**Example 2:** Coated bilayer tablets

The following excipients were used to manufacture the formulations:

Stearin, L2SM® - Cognis Iberia, Castellbisbal, Spain; and
Basic butylated methacrylate copolymer, Eudragit® EPO- Röhm & Haas GmbH, Darmstadt, Germany.

The bilayer tablets obtained in Example 1 were coated such as to protect them from moisture and light, and to improve their organoleptic characteristics (taste masking).

Composition of the formulation used for the coating

| Polymer suspension | |
|---|---|
| Eudragit® EPO | 22.62 g |
| Sodium lauryl sulphate | 2.26 g |
| Stearin L2SM® | 3.39 g |
| Purified water | 160.5 g |
| Pigment suspension | |
| Talc | 5.85 g |

(continued)

| Pigment suspension | |
| --- | --- |
| Titanium dioxide | 1.80 g |
| Magnesium stearate | 7.92 g |
| Purified water | 75.90 g |

Method for manufacturing the coating suspension

Coating conditions used:

| | |
| --- | --- |
| Inlet air temperature | 45˚C |
| Exhaust air temperature | 35 ˚C |
| Peristaltic pump speed | 22 rpm |
| Drum speed | Position 2-3 |
| Atomization pressure | 1.5-2.0 bar |
| Atomization nozzle diameter | 0.8 mm |
| Subsequent drying in air drying chamber | 16 h at 40˚C |
| Tablet weight increase | 5% |

In order to prepare the polymer suspension, sodium lauryl sulphate was added to purified water until diluted, by means of anchor stirring. The stearin was then dispersed and finally the Eudragit® EPO polymer. Total preparation time was 15 minutes.

In order to prepare the pigment suspension, the talc, titanium dioxide and magnesium stearate were added in this order on the purified water with the aid of magnetic stirring. Total preparation time was 15 minutes.

Having prepared the two dispersions, the pigment suspension was added on the polymer suspension, stirried with a turbo-stirrer at an average speed for 30 final minutes.

The coated bilayer tablets obtained showed resistance to the mechanical abrasion caused by the coating system itself due to the friction between the tablets and between the tablets and the inner surface of the coating drum. The coated bilayer tablets gave the same results for the pharmacotechnical and release parameters of each one of the active ingredients as the uncoated bilayer tablets. The assessment and uniformity of content for each one of the active ingredients was comprised between 90 and 110%.

**Example 3:** Methodology for determining pharmacokinetic parameters

1. Uniformity of mass: The AG245 (Mettler-Toledo, Columbus, USA) analytical balance was used for this test. 20 tablets were taken and weighed independently, and the mean, the standard deviation and the variation coefficient of the weights obtained were calculated. A standard deviation of 7.5% was allowed for 180 mg tablets, and of 5.0% for 360 mg tablets.

2. Tablet strength: The PTB-311 (Pharma Test, Hainburg, Germany) durometer was used for this test. Ten tablets were taken to measure tablet hardness and the individual values were recorded, calculating the mean breaking strength, the standard deviation and the variation coefficient.

3. Friability: The TAR10 (Enrveka GmbH, Austria) friabilimeter was used for this test. Twenty tablets were taken and weighed with the AG245 analytical balance, said weight was registered and they were introduced in the friabilimeter for 5 minutes at 25 rpm. After that time, the tablets were recovered from inside the machine, cleaned of possible powder particles that may have been generated by abrasion and they were weighed again with the same analytical balance. Friability was calculated by the difference between the initial weight and the final weight in percentage, this being less than 1 %.

4. Disintegration: This test was performed with the PTZ-E (Pharma Test, Hainburg, Germany) disintegrator, taking 6 tablets and introducing them in each one of the compartments existing in the basket to this effect. The test was performed in a water bath at 37 $\pm$ 1 ˚C and using purified water at the same temperature as a disintegration medium. The mean, standard deviation and variation coefficient were calculated.

5. Loss on drying: The test was performed in an MA-30 thermobalance (Sartorius, Goettingen, Germany). Three tablets were grounded in a glass mortar and the powder obtained was weighed on the thermobalance pan. The process ended after 60 minutes at a temperature of 90 ˚C, the weight loss indicating the percentage of water content.

6. Tablet size: The test was performed with Vernier calipers in order to measure the diameter and height of 10 tablets. The mean, standard deviation and variation coefficient were calculated.

7. Slip velocity and angle of repose: They were determined in a PTG machine (Pharma Test, Hainburg, Germany). Slip velocity measures the capacity of a powder or granules to flow vertically under certain circumstances. The angle of repose is a feature related to friction between particles or resistance to movement between particles. Thus, the instrument provided the reading of both parameters directly after filling the funnel of said machine with the mixture to be tested. The mixture flowed through the funnel forming a cone at the base of the machine, and the time taken to slide through the funnel was measured (slip velocity), as well as the height and diameter of the cone formed at the base, thus being able to calculate the angle of repose ($\alpha$) according to the following equation:

$$\tan(\alpha) = \text{Cone height} / 0.5 \times \text{base diameter}$$

8. Bulk and tapped density: It was performed in STAV 2003 (J. Engelsmann, Ludwigshafen, Germany) titrimetric equipment. A sufficient amount of the mixture was used in the graduated cylinder of the machine to reach a volume of 50-100 mL, weighing the mass of the volume tested. The volume of the mass was recorded before and after tapping. Bulk density was calculated by dividing the mass by the volume before tapping and tapped density by dividing the mass by the volume after tapping. Both densities are expressed in mg/mL.

9. Carr Index (Compressibility factor) and Hausner Index: Both indexes were calculated from bulk density (pa) and tapped bulk density (pac) according to the following equations:

$$\text{Compressibility factor (\%)} = 100 \times (\rho ac - \rho a) / \rho ac$$

$$\text{Hausner Index} = \rho ac / \rho a$$

**Example 4:** Analytical Methods

Solution test

Simvastatin

| Equipment | USP II, paddles |
|---|---|
| Bath temperature | 37˚C $\pm$ 0.5˚C |
| Solution medium | SR 10 mM sodium phosphate at pH 7.0 with sodium lauryl sulphate at 0.5% |
| Average solution volume | 900 mL |
| Paddle stirring rate | 100 rpm |
| Samples | 6 individual tablets (1 for each vessel) |
| Sampling times | Solution kinetics (minimum 3 points) |
| Sampling volume | 5.0 mL, with medium replacement |

(continued)

| Sample filtration | PVDF 0.45 $\mu$m filters |
|---|---|
| Sample analyses | HPLC (without prior sample dilution) |
| Primary data | Area calculation by peak integration |

| Calculations | $$Q = \left[C_n V + \sum_{i=1}^{n-1} C_i S\right]$$<br><br>Wherein:<br>Q = Accumulated dissolved amount of simvastatin (mg)<br>$C_n$ = Simvastatin concentration at sampling interval n (mg/mL)<br>V = Individual volume of machine vessels (mL)<br>$\sum_{i=1}^{n-1} C_i$ = Total simvastatin concentrations sampling interval 1 at n-1 (mg/mL)<br>S = Sampling aliquot volume |
|---|---|

| Chromatographic conditions | |
|---|---|
| Equipment | Kontron Instruments, Bletchley, UK |
| Column | Tracer Excel (Sant Cugat, Spain) 120 ODS 5 $\mu$m 15x0.4 cm (with guard column) |
| Mobile phase | ACN:SR 0.033 M sodium phosphate anhydrous at pH 4.5 65:35 |
| Flow | 1 mL/min |
| Injection volume | 50 $\mu$L 50 $\mu$L |
| Detector | UV, 240 nm |
| Column temperature | TA |
| Total chromatogram time | 18 min |
| Active ingredient retention time | 10 min |

Lisinopril dihydrate

| Equipment | USP II, paddles |
|---|---|
| Bath temperature | 37˚C±0.5˚C |
| Solution medium | SR 30 mM potasium phosphate at pH 2.0 |
| Average solution volume | 500 mL |
| Paddle stirring rate | 100 rpm |
| Samples | 6 individual tablets (1 for each vessel) |
| Sampling times | Solution kinetics (minimum 3 points) |
| Sampling volume | 5.0 mL, with medium replacement |
| Sample filtration | PVDF 0.45 $\mu$m filters |

(continued)

| Sample analyses | HPLC (without prior sample dilution) |
|---|---|
| Primary data | Area calculation by peak integration |
| Calculations | As for simvastatin, accumulated dissolved amounts were calculated and the release percentage was represented graphically. |

| Chromatographic conditions | |
|---|---|
| Equipment | Kontron Instruments |
| Column | Tracer Excel C8 5μm 15x0.4 cm (with guard column) |
| Mobile phase | ACN:SR 0.03 M Sodium phosphate anhydrous at pH 2.0 9:91 |
| Flow | 1 mL/min |
| Injection volume | 50 μL |
| Detector | UV, 215 nm |
| Column temperature | TA |
| Total chromatogram time | 18 min |
| Active ingredient retention time | 8 min |

Folic acid

| Equipment | USP II, paddles |
|---|---|
| Bath temperature | 37 or ± 0.5 ˚C |
| Solution medium | 0.5 M sodium acetate at pH 6.0 |
| Average solution volume | 250 mL |
| Paddle stirring rate | 50 rpm |
| Samples | 6 individual tablets (1 for each vessel) |
| Sampling times | Solution kinetics (minimum 3 points) |
| Sampling volume | 5.0 mL, with medium replacement |
| Sample filtration | PVDF 0.45 μm filters |
| Sample analyses | HPLC (without prior sample dilution) |
| Primary data | Area calculation by peak integration |
| Calculations | As for simvastatin, accumulated dissolved amounts were calculated and the release percentage was represented graphically. |

| Chromatographic conditions | |
|---|---|
| Equipment | Kontron Instruments |
| Column | Tracer Excel 120 ODS 5μm 15x0.4 cm (with guard column) |
| Mobile phase | Methanol:SR 0.03 M Sodium phosphate anhydrous at pH 2.0 22:78 |
| Flow | 1 mL/min |

(continued)

| Chromatographic conditions | |
|---|---|
| Injection volume | 80 $\mu$L |
| Detector | UV, 280 nm |
| Column temperature | TA |
| Total chromatogram time: | 20 min |
| Active ingredient retention time | 9 min |

<u>Assay and uniformity of content</u>

<u>Simvastatin</u>

<u>Assay</u>: Three tablets were individually weighed in different 100 mL flasks and 90 mL of mobile phase were added. These were sonicated for 5 minutes, allowed to cool and brought up to 100 mL with mobile phase. Two mL of the solution were taken and brought to a volume of 10 mL with mobile phase, reaching a final concentration of 20 $\mu$g/mL. The solution was filtered through PVDF 0.45 $\mu$m filters and placed in an HPLC vial.

<u>Uniformity of content</u>: Five tablets were grounded and an equivalent amount of mixture to 5 mg of active ingredient was weighed, and dissolved in about 40 mL of mobile phase. The solution was sonicated for 5 minutes, allowed to cool and brought up to 50 mL with mobile phase. Two mL of the solution were taken and brought to a volume of 10 mL with mobile phase, reaching a final concentration of 20 $\mu$g/mL. This solution was filtered through PVDF 0.45 $\mu$m filters and put into an HPLC vial.

<u>Lisinopril dihydrate</u>

<u>Assay</u>: Three tablets were individually weighed in different 50 mL flasks containing 40 mL of mobile phase. These were sonicated for 5 minutes, allowed to cool and brought up to 50 mL with mobile phase. Two mL of the solution were taken and brought to a volume of 10 mL with mobile phase, reaching a final concentration of 20 $\mu$g/mL. The solution was filtered through PVDF 0.45 $\mu$m filters and placed in an HPLC vial.

<u>Uniformity of content</u>: Five tablets were grounded and the equivalent amount of mixture to 1 mg of active ingredient was weighed, and dissolved in about 20 mL of mobile phase. The solution was sonicated for 5 minutes, allowed to cool and brought up to 25 mL with mobile phase. Two mL of the solution were taken and brought to a volume of 10 mL with mobile phase, reaching a final concentration of 20 $\mu$g/mL. This was filtered through PVDF 0.45 $\mu$m filters and placed in an HPLC vial.

<u>Folic acid</u>

<u>Assay</u>: Three tablets were individually weighed in different 100 mL flasks and 90 mL of mobile phase were added. The solution was sonicated for 5 minutes, allowed to cool and brought up to 100 mL with mobile phase. Final concentration was 4 $\mu$g/mL. This was filtered through PVDF 0.45 $\mu$m filters and placed in an HPLC vial.

<u>Uniformity of content</u>: Five tablets were grounded and 55 mg of the mixture were taken (55.55 $\mu$g p.a.) and dissolved in 20 mL of mobile phase. The solution was sonicated for 5 minutes, allowed to cool and brought up to 25 mL. The final concentration was 4.44 $\mu$g/mL. This was filtered through PVDF 0.45 $\mu$m filters and placed in an HPLC vial.

**Example 5**: Active ingredient release profiles

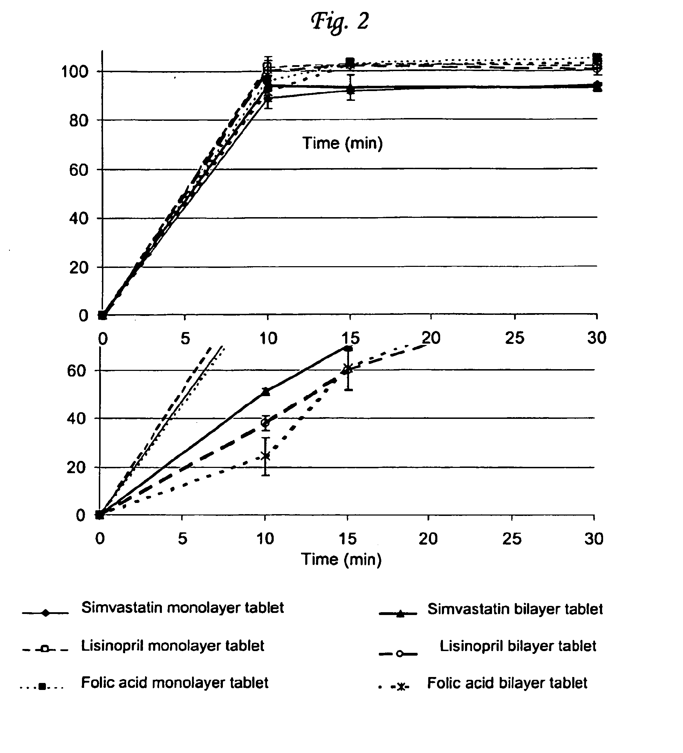

Figure 2 shows the *in vitro* release profile for each one of the active ingredients of the bilayer tablet obtained by direct compression and of the active ingredients separately corresponding to each compartment. The solution rates observed for each one of the active ingredients of the bilayer tablets are in the same order as those of the separate active ingredients.

Figure 3 shows the *in vitro* release profile for each one of the active ingredients of the bilayer tablet obtained by wet granulation and of the active ingredients separately corresponding to each compartment. In this case it is worth mentioning the slowing down of the solution rates observed for each one of the active ingredients of the bilayer tablets with respect to the active ingredients independently, even so releasing the entire doses after 30 minutes.

**Claims**

1. A bilayer tablet for preventing cardiovascular events comprising the following two compartments:

> - a compartment (i) comprising as an active ingredient a pharmaceutically acceptable simvastatin compound; and
> - a compartment (ii) comprising as active ingredients a pharmaceutically acceptable lisinopril compound and a pharmaceutically acceptable folic acid compound;

> **characterized by** the fact that both compartments are isolated from one another.

2. A tablet according to claim 1, **characterized by** the fact that the pharmaceutically acceptable simvastatin compound is free simvastatin, the pharmaceutically acceptable lisinopril compound is chosen from free lisinopril and lisinopril dihydrate and the pharmaceutically acceptable folic acid compound is chosen from free folic acid and folic acid dihydrate.

3. A tablet according to claim 2, **characterized by** the fact that free simvastatin is present in an amount comprised between 2.5 and 20 mg, both inclusive, lisinopril dihydrate or free lisinopril is present in an amount comprised between 1 and 10 mg, both inclusive, and free folic acid is present in an amount comprised between 0.1 and 1 mg, both inclusive; or free simvastatin is present in an amount comprised between 5 and 10 mg, both inclusive, lisinopril dihydrate or free lisinopril is present in an amount comprised between 2.5 and 5 mg, both inclusive, and free folic acid is present in an amount comprised between 0.2 and 0.5 mg, both inclusive.

4. A tablet according to any one of claims 1 to 3, **characterized by** the fact that it is obtained by direct compression and

> - compartment (i) further comprises as excipients a disintegrant agent, a glidant agent, a diluent agent, a lubricant agent and a release agent; and
> - compartment (ii) further comprises as excipients a diluent agent, a disintegrant agent, a glidant agent and a release agent.

5. A tablet according to claim 4, **characterized by** the fact that:

> - the disintegrant agent of compartment (i) is chosen from croscarmellose sodium, sodium starch glycolate, crospovidone, sodium lauryl sulphate, microcrystalline cellulose PH 101, microcrystalline cellulose PH 102 and mixtures thereof, such that the total weight of the disintegrant agents is comprised between 2 and 10% of the compartment weight, both inclusive;
> - the glidant agent of compartment (i) is chosen from colloidal anhydrous silica, corn starch, talc, magnesium trisilicate and mixtures thereof, such that the total weight of the glidant agents is comprised between 0.1 and 10% of the compartment weight, both inclusive;
> - the diluent agent of compartment (i) is chosen from silicified microcrystalline cellulose, microcrystalline cellulose PH 101, microcrystalline cellulose PH 102, anhydrous lactose, hydrated lactose, calcium phosphate, dicalcium phosphate, tricalcium phosphate, mannitol, sorbitol, sucrose, trehalose, xylitol, corn starch, kaolin, bentonite, and mixtures thereof, such that the total weight of the diluent agents is comprised between 20 and 90% of the compartment weight, both inclusive;
> - the lubricant agent of compartment (i) is chosen from talc, sodium benzoate, poloxamer and mixtures thereof, such that the total weight of the lubricant agents is comprised between 1 and 10% of the compartment weight, both inclusive;
> - the release agent of compartment (i) is chosen from magnesium stearate, sodium stearyl fumarate, calcium stearate, zinc stearate, stearic acid, polyethylene glycols with a molecular weight of $\geq 6000$, and mixtures thereof, such that the total weight of the release agents is comprised between 0.25 and 5% of the weight of the compartment, both inclusive;
> - the diluent agent of compartment (ii) is chosen from silicified microcrystalline cellulose, microcrystalline cellulose PH 101, microcrystalline cellulose PH 102, anhydrous lactose, hydrated lactose, calcium phosphate, dicalcium phosphate, tricalcium phosphate, mannitol, sorbitol, sucrose, inositol, trehalose, xylitol, corn starch, kaolin, bentonite and similar agents and mixtures thereof, such that the total weight of the diluent agents is comprised between 20 and 95% of the compartment weight, both inclusive;
> - the glidant agent of compartment (ii) is chosen from colloidal anhydrous silica, corn starch, talc, magnesium trisilicate and mixtures thereof, such that the total weight of the glidant agents is comprised between 0.1 and 10% of the compartment weight, both inclusive;

- the disintegrant agent of compartment (ii) is chosen from croscarmellose sodium, sodium starch glycolate, crospovidone, sodium lauryl sulphate, microcrystalline cellulose PH 101, microcrystalline cellulose PH 102, and mixtures thereof, such that the total weight of the disintegrant agents is comprised between 2 and 5% of the compartment weight, both inclusive;

- the release agent of compartment (ii) is chosen from magnesium stearate, sodium stearyl fumarate, calcium stearate, zinc stearate, stearic acid, polyethylene glycols with a molecular weight of $\geq 6000$, and mixtures thereof.

**6.** A tablet according to any one of claims 1 to 3, **characterized by** the fact that it is obtained by wet granulation and:

- compartment (i) further comprises as excipients a disintegrant agent, a glidant agent, a diluent agent, a binding agent, a solubilizing agent, a lubricant agent and a release agent; and

- compartment (ii) further comprises as excipients a disintegrant agent, a glidant agent, a diluent agent, a binding agent and a release agent.

**7.** A tablet according to claim 6, **characterized by** the fact that:

- the disintegrant agent of compartment (i) is chosen from crospovidone, croscarmellose sodium, sodium starch glycolate, sodium lauryl sulphate, microcrystalline cellulose PH 101, microcrystalline cellulose PH 102 and mixtures thereof, such that the total weight of the disintegrant agents is comprised between 2 and 10% of the compartment weight, both inclusive;

- the glidant agent of compartment (i) is chosen from colloidal anhydrous silica, corn starch, talc, magnesium trisilicate and mixtures thereof, such that the total weight of the glidant agents is comprised between 0.1 and 10% of the compartment weight, both inclusive;

- the diluent agent of compartment (i) is chosen from silicified microcrystalline cellulose, microcrystalline cellulose PH 101, microcrystalline cellulose PH 102, anhydrous lactose, hydrated lactose, calcium phosphate, dicalcium phosphate, tricalcium phosphate, mannitol, sorbitol, sucrose, trehalose, xylitol, corn starch, kaolin, bentonite, and mixtures thereof, such that the total weight of the diluent agents is comprised between 20 and 90% of the compartment weight, both inclusive;

- the binding agent of compartment (i) is chosen from povidone k-30, hydroxypropylmethylcellulose, carboxymethylcellulose, pregelatinized starch, corn starch paste and mixtures thereof such that the total weight of the binding agents is comprised between 0.5 and 20% of the compartment weight, both inclusive;

- the solubilizing agent of compartment (i) is chosen from sodium lauryl sulphate, polysorbate 80, lauroyl macrogol-32 glycerides and mixtures thereof, such that the total weight of the solubilizing agents is comprised between 0.1 and 3% of the compartment weight, both inclusive;

- the lubricant agent of compartment (i) is chosen from talc, sodium benzoate, poloxamer and mixtures thereof, such that the total weight of the lubricant agents is comprised between 1 and 10% of the compartment weight, both inclusive;

- the release agent of compartment (i) is chosen from magnesium stearate, sodium stearyl fumarate, calcium stearate, zinc stearate, stearic acid, polyethylene glycols with a molecular weight of $\geq 6000$ and mixtures thereof, such that the total weight of the release agents is comprised between 0.25 and 5% of the weight of the compartment, both inclusive;

- the disintegrant agent of compartment (ii) is chosen from crospovidone, croscarmellose sodium, sodium starch glycolate, sodium lauryl sulphate, microcrystalline cellulose PH 101, microcrystalline cellulose PH 102 and mixtures thereof, such that the total weight of the disintegrant agents is comprised between 2 and 5% of the compartment weight, both inclusive;

- the glidant agent of compartment (ii) is chosen from colloidal anhydrous silica, corn starch, talc, magnesium trisilicate and mixtures thereof, such that the total weight of the glidant agents is comprised between 0.1 and 10% of the compartment weight, both inclusive;

- the diluent agent of compartment (ii) is chosen from microcrystalline cellulose PH 101, microcrystalline cellulose PH 102 and mixtures thereof, such that the total weight of the diluent agents is comprised between 20 and 50% of the compartment weight, both inclusive;

- the binding agent of compartment (ii) is chosen from povidone k-30, hydroxypropylmethylcellulose, carboxymethylcellulose, pregelatinized starch, corn starch paste and mixtures thereof such that the total weight of the binding agents is comprised between 0.5 and 20% of the compartment weight, both inclusive;

- the release agent of compartment (ii) is chosen from magnesium stearate, sodium stearyl fumarate, calcium stearate, zinc stearate, stearic acid, polyethylene glycols with a molecular weight of $\geq 6000$, and mixtures thereof, such that the total weight of the release agents is comprised between 0.25 and 5 % of the compartment weight, both inclusive.

8. A tablet according to any of claims 1 to 7, **characterized by** the fact that it further comprises a protective coating protecting it from light and moisture.

9. A tablet according to claim 8, wherein the protective coating against light and moisture is performed by means of coating polymers.

10. A tablet according to claim 9, **characterized in that** the coating polymers are chosen from an acrylic polymer and a cellulose derivative.

11. A tablet according to claim 10, **characterized in that** the acrylic polymer is the basic butylated methacrylate copolymer, or
the cellulose derivative is chosen from hydroxypropylmethylcellulose, hydroxypropylcellulose, methylcellulose, hydroxyethylcellulose and mixtures thereof.

12. A tablet according to claim 11, **characterized by** the fact that it optionally further comprises one or more lubricants, one or more plasticizers, or one or more opacifiers.

13. A tablet according to any of the preceding claims, **characterized by** the fact that it has a total weight comprised between 150 and 400 mg, both inclusive, or between 180 and 380 mg, both inclusive.

14. The use of a tablet according to any of claims 1 to 13 for the manufacture of a drug for the prevention of stroke in high-risk conditions or diseases.

15. The use according to claim 14 in which the high-risk conditions or diseases are being older than 55 years of age, angina pectoris, ictus, arteriosclerosis, intermittent claudication, diabetes, coronary disease, peripheral vascular disease, altered platelet function, hemodialysis, hypercholesterolemia, arterial hypertension, myocardial infarction, congestive heart failure, ischaemia, nephropathy, high serum homocysteine levels, cardiac arrest or restenosis, smoking, obesity and a sedentary lifestyle.

**Patentansprüche**

1. Zweischichttablette zur Prävention kardiovaskulärer Anfälle, das die folgenden zwei Kompartimente umfasst:

    - ein Kompartiment (i), das eine pharmazeutisch verträgliche Simvastatin-Verbindung als Wirkstoff umfasst, und
    - ein Kompartiment (ii), das eine pharmazeutisch verträgliche Lisinopril-Verbindung und eine pharmazeutisch verträgliche Folsäureverbindung als Wirkstoffe umfasst;

    **dadurch gekennzeichnet, dass** beide Kompartimente voneinander getrennt sind.

2. Tablette nach Anspruch 1, **dadurch gekennzeichnet, dass** die pharmazeutisch verträgliche Simvastatin-Verbindung freies Simvastatin ist, die pharmazeutisch verträgliche Lisinopril-Verbindung unter freiem Lisinopril und Lisinoprildihydrat ausgewählt ist und die pharmazeutisch verträgliche Folsäureverbindung unter freier Folsäure und Folsäuredihydrat ausgewählt ist.

3. Tablette nach Anspruch 2, **dadurch gekennzeichnet, dass** freies Simvastatin in einer Menge von 2,5 bis 20 mg vorliegt, Lisinoprildihydrat oder freies Lisinopril in einer Menge von 1 bis 10 mg vorliegt und freie Folsäure in einer Menge von 0,1 bis 1 mg vorliegt; oder freies Simvastatin in einer Menge von 5 bis 10 mg vorliegt, Lisinoprildihydrat oder freies Lisinopril in einer Menge von 2,5 bis 5 mg vorliegt und freie Folsäure in einer Menge von 0,2 bis 0,5 mg vorliegt.

4. Tablette nach einem der Ansprüche 1 bis 3, **dadurch gekennzeichnet, dass** sie durch Direktverpressung erhalten wird und

    - Kompartiment (i) ferner ein Zerfallhilfsmittel, ein Gleitmittel, ein Verdünnungsmittel, ein Lubrikans und ein Trennmittel als Exzipient umfasst und
    - Kompartiment (ii) ferner ein Verdünnungsmittel, ein Zerfallhilfsmittel, ein Gleitmittel und ein Trennmittel als Exzipient umfasst.

**5.** Tablette nach Anspruch 4, **dadurch gekennzeichnet, dass**:

- das Zerfallhilfsmittel von Kompartiment (i) ausgewählt ist unter Croscarmellose-Natrium, Natriumstärkeglykolat, Crospovidon, Natriumlaurylsulfat, mikrokristalliner Cellulose PH 101, mikrokristalliner Cellulose PH 102 und Gemischen davon, so dass das Gesamtgewicht der Zerfallhilfsmittel von 2 bis 10 % des Gewichts des Kompartiments liegt;
- das Gleitmittel von Kompartiment (i) ausgewählt ist unter kolloidalem wasserfreiem Siliziumdioxid, Maisstärke, Talk, Magnesiumtrisilikat und Gemischen davon, so dass das Gesamtgewicht der Gleitmittel von 0,1 bis 10 % des Gewichts des Kompartiments liegt;
- das Verdünnungsmittel von Kompartiment (i) ausgewählt ist unter silizifizierter mikrokristalliner Cellulose PH 101, mikrokristalliner Cellulose PH 102, wasserfreier Laktose, wasserhaltiger Laktose, Calciumphosphat, Dicalciumphosphat, Tricalciumphosphat, Mannit, Sorbit, Saccharose, Trehalose, Xylit, Maisstärke, Kaolin, Bentonit und Gemischen davon, so dass das Gesamtgewicht der Verdünnungsmittel von 20 bis 90 % des Gewichts des Kompartiments liegt;
- das Lubrikans von Kompartiment (i) ausgewählt ist unter Talk, Natriumbenzoat, Poloxamer und Gemischen davon, so dass das Gesamtgewicht der Lubricanzien von 1 bis 10 % des Gewichts des Kompartiments liegt;
- das Trennmittel von Kompartiment (i) ausgewählt ist unter Magnesiumstearat, Natriumstearylfumarat, Calciumstearat, Zinkstearat, Stearinsäure, Polyethylenglykolen mit einem Molekulargewicht von $\geq$ 6000 und Gemischen davon, so dass das Gesamtgewicht der Trennmittel von 0,25 bis 5 % des Gewichts des Kompartiments liegt;
- das Verdünnungsmittel von Kompartiment (ii) ausgewählt ist unter silizifizierter mikrokristalliner Cellulose, mikrokristalliner Cellulose PH 101, mikrokristalliner Cellulose PH 102, wasserfreier Laktose, wasserhaltiger Laktose, Calciumphosphat, Dicalciumphosphat, Tricalciumphosphat, Mannit, Sorbit, Saccharose, Inositol, Trehalose, Xylit, Maisstärke, Kaolin, Bentonit und ähnlichen Agenzien und Gemischen davon, so dass das Gesamtgewicht der Verdünnungsmittel von 20 bis 95 % des Gewichts des Kompartiments liegt;
- das Gleitmittel von Kompartiment (ii) ausgewählt ist unter kolloidalem wasserfreiem Siliciumdioxid, Maisstärke, Talk, Magnesiumtrisilikat und Gemischen davon, so dass das Gesamtgewicht der Gleitmittel von 0,1 bis 10 % des Gewichts des Kompartiments liegt;
- das Zerfallhilfsmittel von Kompartiment (ii) ausgewählt ist unter Croscarmellose-Natrium, Natriumstärkeglykolat, Crospovidon, Natriumlaurylsulfat, mikrokristalliner Cellulose PH 101, mikrokristalliner Cellulose PH 102 und Gemischen davon, so dass das Gesamtgewicht der Zerfallhilfsmittel von 2 bis 5 % des Gewichts des Kompartiments liegt;
- das Trennmittel von Kompartiment (ii) ausgewählt ist unter Magnesiumstearat, Natriumstearylfumarat, Calciumstearat, Zinkstearat, Stearinsäure, Polyethylenglykolen mit einem Molekulargewicht von $\geq$ 6000 und Gemischen davon.

**6.** Tablette nach einem der Ansprüche 1 bis 3, **dadurch gekennzeichnet, dass** sie durch Nassgranulierung erhalten wird und:

- Kompartiment (i) ferner ein Zerfallhilfsmittel, ein Gleitmittel, ein Verdünnungsmittel, ein Bindemittel, ein Solubilisierungsmittel, ein Lubrikans und ein Trennmittel als Arzneimittelträger umfasst und
- Kompartiment (ii) ferner ein Zerfallhilfsmittel, ein Gleitmittel, ein Verdünnungsmittel, ein Bindemittel und ein Trennmittel als Arzneimittelträger umfasst.

**7.** Tablette nach Anspruch 6, **dadurch gekennzeichnet, dass**:

- das Zerfallhilfsmittel von Kompartiment (i) ausgewählt ist unter Crospovidon, Croscarmellose-Natrium, Natriumstärkeglykolat, Natriumlaurylsulfat, mikrokristalliner Cellulose PH 101, mikrokristalliner Cellulose PH 102 und Gemischen davon, so dass das Gesamtgewicht der Zerfallhilfsmittel von 2 bis 10 % des Gewichts des Kompartiments liegt;
- das Gleitmittel von Kompartiment (i) ausgewählt ist unter kolloidalem wasserfreiem Siliziumdioxid, Maisstärke, Talk, Magnesiumtrisilikat und Gemischen davon, so dass das Gesamtgewicht der Gleitmittel von 0,1 bis 10 % des Gewichts des Kompartiments liegt;
- das Verdünnungsmittel von Kompartiment (i) ausgewählt ist unter silizifizierter mikrokristalliner Cellulose, mikrokristalliner Cellulose PH 101, mikrokristalliner Cellulose PH 102, wasserfreier Laktose, wasserhaltiger Laktose, Calciumphosphat, Dicalciumphosphat, Tricalciumphosphat, Mannit, Sorbit, Saccharose, Trehalose, Xylit, Maisstärke, Kaolin, Bentonit und Gemischen davon, so dass das Gesamtgewicht der Verdünnungsmittel von 20 bis 90 % des Gewichts des Kompartiments liegt;

- das Bindemittel von Kompartiment (i) ausgewählt ist unter Povidon K-30, Hydroxypropylmethylcellulose, Carboxymethylcellulose, vorverkleisterter Stärke, verkleisterter Maisstärke und Gemischen davon, so dass das Gesamtgewicht der Bindemittel von 0,5 bis 20 % des Gewichts des Kompartiments liegt;

- das Solubilisierungsmittel von Kompartiment (i) ausgewählt ist unter Natriumlaurylsulfat, Polysorbat 80, Lauroyl-Macrogol-32-Glyceriden und Gemischen davon, so dass das Gesamtgewicht der Solubilisierungsmittel von 0,1 bis 3 % des Gewichts des Kompartiments liegt;

- das Lubrikans von Kompartiment (i) ausgewählt ist unter Talk, Natriumbenzoat, Poloxamer und Gemischen davon, so dass das Gesamtgewicht der Lubrikanzien von 1 bis 10 % des Gewichts des Kompartiments liegt;

- das Trennmittel von Kompartiment (i) ausgewählt ist unter Magnesiumstearat, Natriumstearylfumarat, Calciumstearat, Zinkstearat, Stearinsäure, Polyethylenglykolen mit einem Molekulargewicht von ≥6000 und Gemischen davon, so dass das Gesamtgewicht der Trennmittel von 0,25 bis 5 % des Gewichts des Kompartiments liegt;

- das Zerfallhilfsmittel von Kompartiment (ii) ausgewählt ist unter Crospovidon, Croscarmellose-Natrium, Natriumstärkeglykolat, Natriumlaurylsulfat, mikrokristalliner Cellulose PH 101, mikrokristalliner Cellulose PH 102 und Gemischen davon, so dass das Gesamtgewicht der Zerfallhilfsmittel von 2 bis 5 % des Gewichts des Kompartiments liegt;

- das Gleitmittel von Kompartiment (ii) ausgewählt ist unter kolloidalem wasserfreiem Siliziumdioxid, Maisstärke, Talk, Magnesiumtrisilikat und Gemischen davon, so dass das Gesamtgewicht der Gleitmittel von 0,1 bis 10 % des Gewichts des Kompartiments liegt;

- das Verdünnungsmittel von Kompartiment (ii) ausgewählt ist unter mikrokristalliner Cellulose PH 101, mikrokristalliner Cellulose PH 102 und Gemischen davon, so dass das Gesamtgewicht der Verdünnungsmittel von 20 bis 50 % des Gewichts des Kompartiments liegt;

- das Bindemittel von Kompartiment (ii) ausgewählt ist unter Povidon K-30, Hydroxypropylmethylcellulose, Carboxymethylcellulose, vorverkleisterter Stärke, verkleisterter Maisstärke und Gemischen davon, so dass das Gesamtgewicht der Bindemittel von 0,5 bis 20 % des Gewichts des Kompartiments liegt;

- das Trennmittel von Kompartiment (ii) ausgewählt ist unter Magnesiumstearat, Natriumstearylfumarat, Calciumstearat, Zinkstearat, Stearinsäure, Polyethylenglykolen mit einem Molekulargewicht von ≥ 6000 und Gemischen davon, so dass das Gesamtgewicht der Trennmittel von 0,25 bis 5 % des Gewichts des Kompartiments liegt.

**8.** Tablette nach einem der Ansprüche 1 bis 7, **dadurch gekennzeichnet, dass** sie ferner einen Schutzüberzug umfasst, der sie vor Licht und Feuchtigkeit schützt.

**9.** Tablette nach Anspruch 8, wobei der Schutzüberzug gegen Licht und Feuchtigkeit mit Hilfe von Beschichtungspolymeren erfolgt.

**10.** Tablette nach Anspruch 9, **dadurch gekennzeichnet, dass** die Beschichtungspolymere unter einem Acrylpolymer und einem Cellulosederivat ausgewählt sind.

**11.** Tablette nach Anspruch 10, **dadurch gekennzeichnet, dass** das Acrylpolymer das basische butylierte Methacrylatcopolymer ist oder das Cellulosederivat unter Hydroxypropylmethylcellulose, Hydroxypropylcellulose, Methylcellulose, Hydroxyethylcellulose und Gemischen davon ausgewählt ist.

**12.** Tablette nach Anspruch 11, **dadurch gekennzeichnet, dass** sie gegebenenfalls ferner ein oder mehrere Lubrikanzien, einen oder mehrere Weichmacher oder ein oder mehrere Trübungsmittel umfasst.

**13.** Tablette nach einem der vorhergehenden Ansprüche, **dadurch gekennzeichnet, dass** sie ein Gesamtgewicht von 150 bis 400 mg oder von 180 bis 380 mg aufweist.

**14.** Verwendung einer Tablette nach einem der Ansprüche 1 bis 13 zur Herstellung eines Arzneimittels zur Prävention von Schlaganfall bei Hochrisikozuständen oder -erkrankungen.

**15.** Verwendung nach Anspruch 14, bei welcher die Hochrisikozustände oder -erkrankungen ein Lebensalter über 55 Jahre, Angina pectoris, Iktus, Arteriosklerose, Claudicatio intermittens, Diabetes, Koronarerkrankung, periphere Gefäßerkrankung, veränderte Plättchenfunktion, Hämodialyse, Hypercholesterinämie, arterielle Hypertonie, Myocardinfarkt, kongestive Herzinsuffizienz, Ischämie, Nephropathie, hohe Homocysteinspiegel im Serum, Herzstillstand oder Restenose, Rauchen, Übergewicht und Bewegungsmangel sind.

**Revendications**

1. Comprimé bicouche pour la prévention d'accidents cardiovasculaires, comprenant les deux compartiments suivants :

    - un compartiment (i) comprenant en tant qu'ingrédient actif un composé de simvastatine pharmaceutiquement acceptable ; et
    - un compartiment (ii) comprenant en tant qu'ingrédients actifs un composé de lisinopril pharmaceutiquement acceptable et un composé d'acide folique pharmaceutiquement acceptable ;

    **caractérisé en ce que** les deux compartiments sont isolés l'un de l'autre.

2. Comprimé selon la revendication 1, **caractérisé en ce que** le composé de simvastatine pharmaceutiquement acceptable est de la simvastatine libre, le composé de lisinopril pharmaceutiquement acceptable est choisi parmi le lisinopril libre et le lisinopril dihydraté, et le composé d'acide folique pharmaceutiquement acceptable est choisi parmi l'acide folique libre et l'acide folique dihydraté.

3. Comprimé selon la revendication 2, **caractérisé en ce que** la teneur en simvastatine libre va de 2,5 à 20 mg, la teneur en lisinopril dihydraté ou en lisinorpil libre va de 1 à 10 mg, et la teneur en acide folique libre va de 0,1 à 1 mg ; ou la teneur en simvastatine libre va de 5 à 10 mg, la teneur en lisinopril dihydraté ou en lisinorpil libre va de 2,5 à 5 mg, et la teneur en acide folique libre va de 0,2 à 0,5 mg.

4. Comprimé selon l'une quelconque des revendications 1 à 3, **caractérisé en ce qu'**il est obtenu par compression directe et **en ce que**

    - le compartiment (i) comprend en outre en tant qu'excipients un délitant, un glissant, un diluant, un lubrifiant et un agent de libération ; et
    - le compartiment (ii) comprend en outre en tant qu'excipients un diluant, un délitant, un glissant et un agent de libération.

5. Comprimé selon la revendication 4, **caractérisé en ce que** :

    - le délitant du compartiment (i) est choisi parmi les éléments suivants : croscarmellose sodique, glycolate d'amidon sodique, crospovidone, laurylsulfate de sodium, cellulose microcristalline PH 101, cellulose microcristalline PH 102, et des mélanges de ceux-ci, de telle sorte que le poids total des délitants va de 2 à 10 % du poids du compartiment ;
    - le glissant du compartiment (i) est choisi parmi les éléments suivants : silice colloïdale anhydre, amidon de maïs, talc, trisilicate de magnésium, et des mélanges de ceux-ci, de telle sorte que le poids total des glissants va de 0,1 à 10 % du poids du compartiment ;
    - le diluant du compartiment (i) est choisi parmi les éléments suivants : cellulose microcristalline silicifiée, cellulose microcristalline PH 101, cellulose microcristalline PH 102, lactose anhydre, lactose hydraté, phosphate de calcium, phosphate dicalcique, phosphate tricalcique, mannitol, sorbitol, saccharose, tréhalose, xylitol, amidon de maïs, kaolin, bentonite, et des mélanges de ceux-ci, de telle sorte que le poids total des diluants va de 20 à 90 % du poids du compartiment ;
    - le lubrifiant du compartiment (i) est choisi parmi les éléments suivants : talc, benzoate de sodium, poloxamère, et des mélanges de ceux-ci, de telle sorte que le poids total des lubrifiants va de 1 à 10 % du poids du compartiment ;
    - l'agent de libération du compartiment (i) est choisi parmi les éléments suivants : stéarate de magnésium, stéarylfumarate de sodium, stéarate de calcium, stéarate de zinc, acide stéarique, glycols polyéthyléniques d'un poids moléculaire ≥ 6 000, et des mélanges de ceux-ci, de telle sorte que le poids total des agents de libération va de 0,25 à 5 % du poids du compartiment ;
    - le diluant du compartiment (ii) est choisi parmi les éléments suivants : cellulose microcristalline silicifiée, cellulose microcristalline PH 101, cellulose microcristalline PH 102, lactose anhydre, lactose hydraté, phosphate de calcium, phosphate dicalcique, phosphate tricalcique, mannitol, sorbitol, saccharose, inositol, tréhalose, xylitol, amidon de maïs, kaolin, bentonite, des agents similaires et des mélanges de ceux-ci, de telle sorte que le poids total des diluants va de 20 à 95 % du poids du compartiment ;
    - le glissant du compartiment (ii) est choisi parmi les éléments suivants : silice colloïdale anhydre, amidon de maïs, talc, trisilicate de magnésium, et des mélanges de ceux-ci, de telle sorte que le poids total des glissants va de 0,1 à 10 % du poids du compartiment ;
- le délitant du compartiment (ii) est choisi parmi les éléments suivants : croscarmellose sodique, glycolate d'amidon sodique, crospovidone, laurylsulfate de sodium, cellulose microcristalline PH 101, cellulose microcristalline PH 102, et des mélanges de ceux-ci, de telle sorte que le poids total des délitants va de 2 à 5 % du poids du compartiment ;
- l'agent de libération du compartiment (ii) est choisi parmi les éléments suivants : stéarate de magnésium, stéarylfumarate de sodium, stéarate de calcium, stéarate de zinc, acide stéarique, glycols polyéthyléniques d'un poids moléculaire ≥ 6 000, et des mélanges de ceux-ci.

6. Comprimé selon l'une quelconque des revendications 1 à 3, **caractérisé en ce qu'**il est obtenu par granulation par voie humide et **en ce que** :

- le compartiment (i) comprend en outre en tant qu'excipients un délitant, un glissant, un diluant, un agglutinant, un solubilisant, un lubrifiant et un agent de libération ; et
- le compartiment (ii) comprend en outre en tant qu'excipients un délitant, un glissant, un diluant, un agglutinant et un agent de libération.

7. Comprimé selon la revendication 6, **caractérisé en ce que**:

- le délitant du compartiment (i) est choisi parmi les éléments suivants : crospovidone, croscarmellose sodique, glycolate d'amidon sodique, laurylsulfate de sodium, cellulose microcristalline PH 101, cellulose microcristalline PH 102, et des mélanges de ceux-ci, de telle sorte que le poids total des délitants va de 2 à 10 % du poids du compartiment ;
- le glissant du compartiment (i) est choisi parmi les éléments suivants : silice colloïdale anhydre, amidon de maïs, talc, trisilicate de magnésium, et des mélanges de ceux-ci, de telle sorte que le poids total des glissants va de 0,1 à 10 % du poids du compartiment ;
- le diluant du compartiment (i) est choisi parmi les éléments suivants : cellulose microcristalline silicifiée, cellulose microcristalline PH 101, cellulose microcristalline PH 102, lactose anhydre, lactose hydraté, phosphate de calcium, phosphate dicalcique, phosphate tricalcique, mannitol, sorbitol, saccharose, tréhalose, xylitol, amidon de maïs, kaolin, bentonite, et des mélanges de ceux-ci, de telle sorte que le poids total des diluants va de 20 à 90 % du poids du compartiment ;
- l'agglutinant du compartiment (i) est choisi parmi les éléments suivants : povidone k-30, hydroxypropylméthylcellulose, carboxyméthyl-cellulose, amidon prégélatinisé, empois d'amidon de maïs et des mélanges de ceux-ci, de telle sorte que le poids total des agglutinants va de 0,5 à 20 % du poids du compartiment ;
- le solubilisant du compartiment (i) est choisi parmi les éléments suivants : laurylsulfate de sodium, polysorbate 80, macrogol-32 glycérides de lauroyle, et des mélanges de ceux-ci, de telle sorte que le poids total des solubilisants va de 0,1 à 3 % du poids du compartiment ;
- le lubrifiant du compartiment (i) est choisi parmi les éléments suivants : talc, benzoate de sodium, poloxamère, et des mélanges de ceux-ci, de telle sorte que le poids total des lubrifiants va de 1 à 10 % du poids du compartiment ;
- l'agent de libération du compartiment (i) est choisi parmi les éléments suivants : stéarate de magnésium, stéarylfumarate de sodium, stéarate de calcium, stéarate de zinc, acide stéarique, glycols polyéthyléniques d'un poids moléculaire à 6 000, et des mélanges de ceux-ci, de telle sorte que le poids total des agents de libération va de 0,25 à 5 % du poids du compartiment ;
- le délitant du compartiment (ii) est choisi parmi les éléments suivants : crospovidone, croscarmellose sodique, glycolate d'amidon sodique, laurylsulfate de sodium, cellulose microcristalline PH 101, cellulose microcristalline PH 102, et des mélanges de ceux-ci, de telle sorte que le poids total des délitants va de 2 à 5 % du poids du compartiment ;
- le glissant du compartiment (ii) est choisi parmi les éléments suivants : silice colloïdale anhydre, amidon de maïs, talc, trisilicate de magnésium, et des mélanges de ceux-ci, de telle sorte que le poids total des glissants va de 0,1 à 10 % du poids du compartiment ;
- le diluant du compartiment (ii) est choisi parmi les éléments suivants : cellulose microcristalline PH 101, cellulose microcristalline PH 102, et des mélanges de ceux-ci, de telle sorte que le poids total des diluants va de 20 à 50 % du poids du compartiment ;
- l'agglutinant du compartiment (ii) est choisi parmi les éléments suivants : povidone k-30, hydroxypropylméthylcellulose, carboxyméthyl-cellulose, amidon prégélatinisé, empois d'amidon de maïs et des mélanges de ceux-ci, de telle sorte que le poids total des agglutinants va de 0,5 à 20 % du poids du compartiment ;
- l'agent de libération du compartiment (ii) est choisi parmi les éléments suivants : stéarate de magnésium, stéarylfumarate de sodium, stéarate de calcium, stéarate de zinc, acide stéarique, glycols polyéthyléniques d'un poids moléculaire ≥ 6 000, et des mélanges de ceux-ci, de telle sorte que le poids total des agents de libération va de 0,25 à 5 % du poids du compartiment.

8. Comprimé selon l'une quelconque des revendications 1 à 7, **caractérisé en ce qu'**il comprend en outre un enrobage protecteur le protégeant de la lumière et de l'humidité.

9. Comprimé selon la revendication 8, dans lequel l'enrobage de protection contre la lumière et l'humidité est formé au moyen de polymères d'enrobage.

10. Comprimé selon la revendication 9, **caractérisé en ce que** les polymères d'enrobage sont choisis parmi un polymère acrylique et un dérivé de cellulose.

11. Comprimé selon la revendication 10, **caractérisé en ce que** le polymère acrylique est le copolymère de butylméthacrylate basique, ou **en ce que** le dérivé de cellulose est choisi parmi les éléments suivantes : hydroxypropylméthylcellulose, hydroxypropylcellulose, méthylcellulose, hydroxyéthyl-cellulose, et des mélanges de ceux-ci.

12. Comprimé selon la revendication 11, **caractérisé en ce qu'**il comprend en outre optionnellement un ou plusieurs lubrifiants, un ou plusieurs plastifiants, un ou plusieurs opacifiants.

13. Comprimé selon l'une quelconque des revendications précédentes, **caractérisé en ce qu'**il a un poids total allant de 150 à 400 mg ou de 180 à 380 mg.

14. Utilisation d'un comprimé selon l'une quelconque des revendications 1 à 13 pour la fabrication d'un médicament destiné à la prévention d'un accident vasculaire cérébral dans des conditions ou pathologies à risque élevé.

15. Utilisation selon la revendication 14, dans laquelle les conditions ou pathologies à risque élevé sont un âge supérieur à 55 ans, l'angine de poitrine, l'ictus apoplectique, l'artériosclérose, la claudication intermittente, le diabète, les maladies coronariennes, les acrosyndromes vasculaires, les troubles de la fonction plaquettaire, l'hémodialyse, l'hypercholestérolémie, l'hypertension artérielle, l'infarctus du myocarde, l'insuffisance cardiaque congestive, l'ischémie, la néphropathie, un taux élevé d'homocystéine sérique, l'arrêt cardiaque ou la resténose, le tabagisme, l'obésité et un mode de vie sédentaire.

# Fig. 1

A = 5.50 mm

B = 2.25 mm

C = 2.25 mm

D = 9.00 mm

# Fig. 2

| | |
|---|---|
| ━◆━ Simvastatin monolayer tablet | ━▲━ Simvastatin bilayer tablet |
| ─-▫-─ Lisinopril monolayer tablet | ─○─ Lisinopril bilayer tablet |
| ···■··· Folic acid monolayer tablet | ·-✕- Folic acid bilayer tablet |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6576256 B
- WO 0115674 A
- WO 0176632 A
- WO 03020243 A
- WO 2004080488 A
- WO 2005011586 A
- WO 2005025673 A

**Non-patent literature cited in the description**

- **Wald ; Law.** *Br. Med. J.,* 2003, vol. 326 (7404), 1419-23